# EUROPEAN PATENT APPLICATION

(11) **EP 4 467 637 A1**
(43) Date of publication of application: **27.11.2024**
(21) Application number: 22921240.2
(22) Date of filing: 09.02.2022
(51) Int. Cl.: C12N 5/07, C12Q 1/02

(54) **CULTURE MEDIUM AND CULTURE METHOD FOR MAMMARY EPITHELIAL CELLS, AND USE OF MAMMARY EPITHELIAL CELLS**

(30) Priority: 19.01.2022 CN 202210058996
(71) Applicant: Precedo Pharmaceuticals Co., Ltd., Hefei, Anhui 230094 (CN)
(72) Inventor: LIU, Qing Song, Hefei, Anhui 230031 (CN); LIU, Fei Yang, Hefei, Anhui 230031 (CN); ZHANG, Jie, Hefei, Anhui 230094 (CN); CHEN, Cheng, Hefei, Anhui 230031 (CN); WANG, Wen Chao, Hefei, Anhui 230031 (CN)
(74) Representative: Abel & Imray LLP
(86) International application number: PCT/CN2022/075575
(87) International publication number: WO 2023/137804

(57) **Abstract**

A culture medium for culturing mammary epithelial cells, which medium contains β-estradiol, insulin-like growth factor 1, basic fibroblast growth factor, tumor necrosis factor-α, and optionally fibroblast growth factor 10. A method for culturing mammary epithelial cells using the culture medium, and a method and the use of cells obtained using the culture method in evaluating or screening the efficacy of a drug.

## Description

### Technical Field

The invention relates to a culture medium for *in vitro* culturing epithelial cells, in particular mammary epithelial cells, and a culture method for culturing the cells or organoids comprising the cells. The invention also relates to the use of cell progeny or organoids cultured by the culture medium and culture method of the invention in efficacy evaluating and screening of drugs, toxicity determination and regenerative medicine.

### Background of the Invention

The *in vitro* culture of human mammary epithelial cells is crucial for studying the mechanisms of normal mammary development and the occurrence and development of mammary tumors. However, there are still many challenges in achieving sustainable culture of human mammary epithelial cells. At present, two renewable technologies have been reported to be used for the *in vitro* culture of human mammary epithelial cells: one is the conditional reprogramming technology developed by Georgetown University in the United States, and the other is the organoid technology developed by the Royal Netherlands Academy of Arts and Sciences. However, there are certain problems in the practical application of these two technologies: the conditional reprogramming technology requires the co-culture of the patient's autologous tumor cells with mouse-derived feeder cells, which may interfere with the analysis results of tumor cells; the culture medium used in the organoid technology takes stem-cell niche factors as the core component, but the amount of niche factors required in this culture technology is large, the cost is high, and the culture cycle is long, which is difficult to operate. Therefore, neither of these two technologies has been really promoted to the field of basic research and new drug development on a large scale.

The inventor has recorded in the patent (WO 2021/088119) a culture medium and culture method for *in vitro* culture of mammary epithelial cells without feeder cells, with controllable cost and convenient operation. This is the first two-dimensional culture system for mammary epithelial stem cells *in vitro* without requiring feeder cells and Wnt agonists such as Wnt protein and proteins of R-spondin family in the culture components. This culture system can maintain the continuous proliferation of mammary epithelial cells *in vitro* for at least 1 month. Based on the original invention, the inventor unexpectedly discovered that the application of additives such as tumor necrosis factor to the culture of mammary epithelial cells can achieve a faster effect of promoting the continuous

### Summary of the Invention

The invention provides a culture medium and a culture method for culturing mammary epithelial cells. The culture medium comprises β-estradiol, insulin-like growth factor 1, basic fibroblast growth factor, tumor necrosis factor-α, and optionally fibroblast growth factor 10.

In an embodiment of the invention, the amount of each component in the culture medium of the invention satisfies any one or more or all of the following conditions:
the concentration of β-estradiol is 5 nM to 50 nM, more preferably 5 nM to 10 nM;
the concentration of insulin-like growth factor 1 is 10 ng/ml to 200 ng/ml, more preferably 20 ng/ml to 100 ng/ml;
the concentration of basic fibroblast growth factor is 10 ng/ml to 200 ng/ml, more preferably 20 ng/ml to 100 ng/ml;
the concentration of tumor necrosis factor-α is 2 ng/ml to 100 ng/ml, more preferably 5 ng/ml to 50 ng/ml; and
the concentration of fibroblast growth factor 10 optionally added is 0 ng/ml to 50 ng/ml.

In an embodiment of the invention, the culture medium of the invention further comprises amphiregulin, epidermal growth factor, insulin, B27, ROCK inhibitor Y27632, neuregulin 1, fibroblast growth factor 7, TGFβ type I receptor inhibitor A8301, and GlutaMAX-I.

Wherein, in a preferred aspect, the amount of amphiregulin is 10 ng/ml to 100 ng/ml; the amount of epidermal growth factor is 2.5 ng/ml to 20 ng/ml; the amount of insulin is 1 µg/ml to 10 µg/ml; B27 is added at a volume ratio of 1:25 to 1:100; the amount of Y27632 is 5 µM to 15 µM; the amount of neuregulin 1 is 5 nM to 20 nM; the amount of fibroblast growth factor 7 is 2.5 ng/ml to 20 ng/ml; the amount of A8301 is 100 nM to 500 nM; and GlutaMAX-I is added at a volume ratio of 1:50 to 1:200.

In an embodiment of the invention, the culture medium further comprises an initial culture medium selected from the group consisting of DMEM/F12, DMEM, F12 or RPMI-1640; and one or more antibiotic(s) selected from the group consisting of streptomycin/penicillin, amphotericin B and Primocin.

In a preferred embodiment, in case of using streptomycin/penicillin as the antibiotic(s), the concentration range of streptomycin is 25-400 µg/mL, the concentration range of penicillin is 25-400 U/mL; in case of using amphotericin B as the antibiotic, the concentration range is 0.25-4 µg/mL; and in case of using Primocin as the antibiotic, the concentration range is 25-400 µg/mL.

A second aspect of the invention provides a culture method for mammary epithelial cells, wherein the culture method comprises the following steps: (1) preparing the culture medium for mammary epithelial cells of the invention; (2) coating a culture vessel with an extracellular matrix gel; (3) inoculating primary mammary epithelial cells in the coated culture vessel and culturing them using the culture medium for mammary epithelial cells of the invention.

Wherein, the extracellular matrix gel used in the culture method is growth factor reduced-type extracellular matrix gel, for example, commercially available Matrigel (Corning: 354230) or BME (Trevigen: 3533-010-02). More specifically, the extracellular matrix gel is diluted with a serum-free culture medium, and the culture medium can be the initial culture medium of the invention, such as DMEM/F12 (Corning: R10-092-CV). The dilution ratio of the extracellular matrix gel is 1:20-400, preferably 1:50-200. The coating method is to add the diluted extracellular matrix gel into a culture vessel so that it completely covers the bottom of the culture vessel, and leave it to coat for more than 30 minutes, preferably at 37°C, and preferably for 30 to 60 minutes. After the coating is completed, the excess extracellular matrix gel dilution is aspirated and discarded, and the culture vessel is ready for use.

The human mammary epithelial cells can be breast cancer tumor cells, normal mammary epithelial cells, or mammary epithelial stem cells. For example, the above tissue samples are collected within half an hour of surgical resection or biopsy of the patient. More specifically, under a sterile environment, a tissue sample of non-necrotic area is cut, the volume of which is 0.5 cm³ or more, and it is placed in a pre-cooled DMEM/F12 culture medium containing antibiotics, and transported to the laboratory on ice; for example, DMEM/F12 culture medium containing 50-200 U/mL (e.g., 100 U/mL) of penicillin and 50-200 µg/mL (e.g., 100 µg/mL) of streptomycin is used for ice transportation.

In a biosafety cabinet, the tissue sample is transferred to a cell culture dish, and then rinsed with the transport fluid. The blood cells on the surface of the tissue sample are washed away, and unnecessary tissues such as skin and fascia on the surface of the tissue sample are removed.

The rinsed tissue sample is transferred to another new culture dish, with the addition of 5-25 mL of the transport fluid. The tissue sample is divided into tissue fragments less than 1 mm³ in diameter using a sterile scalpel blade and a forceps.

The tissue sample fragments are transferred to a centrifuge tube, and centrifuged at 1000 rpm or more for 3-10 minutes in a tabletop centrifuge; after the supernatant is carefully removed from the centrifuge tube with a pipettor, the precipitate is resuspended in 5-25 mL of serum-free DMEM/F12 culture medium containing collagenase II (0.5-5 mg/mL, e.g. 1 mg/mL) and collagenase IV (0.5-5 mg/mL, e.g. 1 mg/mL), and placed in a constant temperature shaker of 37°C for shake digestion for at least 30 minutes (digestion time depends on the sample size; if the sample is larger than 1 g, the digestion time increases to 1.5-2 hours); then it is centrifuged at 300 g/minute or more for 3-10 minutes in a tabletop centrifuge; the supernatant is discarded, and the digested tissue cells are resuspended in 5-25 mL of DMEM/F12 culture medium containing, for example, 10% fetal bovine serum, ground and sieved, with the pore size of the cell sieve of, for example, 100 µm; the sieved cell suspension is collected in centrifuge tubes; and the cells are counted with a hemocytometer.

The cell suspension is then centrifuged at at least 300 g/min for 3-10 minutes in a centrifuge. The supernatant is discarded, and the pellet is resuspended in the culture medium of the invention, and then inoculated into the coated culture vessel at a density of 1×10⁴ to 1 ×10⁶ cells/well for culture.

A third aspect of the invention provides a method for evaluating or screening a drug for treating breast diseases, comprising using the culture medium and culture method of the invention to obtain expanded offspring mammary epithelial cells, and applying the obtained cells to the efficacy evaluation and screening of drug, especially to the *in vitro* efficacy evaluation and screening of anti-tumor drugs.

Preferably, the method for evaluating or screening drugs for treating breast diseases comprises the following steps:
(1) obtaining primary mammary epithelial cells and culturing them using the culture method of the invention;
(2) selecting the drug to be tested and preparing it into different concentration gradients;
(3) adding different concentrations of the drug prepared in step (2) to the mammary epithelial cells cultured in step (1); and
(4) detecting the cell viability.

The beneficial effects of the invention include the following:
(1) rapid expansion of mammary epithelial cells in a short period of time is achieved, and a sufficient number of cells can be obtained within an effective time for application in basic research and drug screening;
(2) mammary epithelial cells obtained by *in vitro* culture using the culture medium and culture method of the invention can maintain the heterogeneity of the patient from whom the cells are derived, and can be applied in the field of regenerative medicine;
(3) the cultured mammary epithelial cells are not interfered by cells such as fibroblasts, and purified mammary epithelial cells and their progeny can be obtained;
(4) the culture medium does not contain any uncertain components such as serum, so it is not affected by the quality and quantity of serum from different batches; and
(5) the technology does not require the addition of expensive stem cell niche factors such as R-spondin and Wnt family components to culture mammary epithelial cells, and compared with the culture medium disclosed in WO 2021/088119, the culture medium of the invention can obtain a large number of monolayer epithelial cells more rapidly, and the cycle of expanding to obtain the same cell number is shorter, which is suitable for application in the fields of efficacy evaluation, screening and toxicity testing of drug, such as high-throughput screening of new candidate compounds and providing high-throughput drug sensitivity function test *in vitro* for patients.

The culture medium of the embodiment can be used to culture mammary epithelial cells derived from humans or other mammals, or tissues containing at least any one of these cells, to obtain expanded, corresponding mammary epithelial cell progeny.

In addition, the cells obtained by the culture method of the embodiment can be applied to regenerative medicine, toxicity testing, basic medical research on mammary epithelial cells, screening of drug responses, determination of *in vitro* metabolic stability and metabolic profile of drugs, and development of new drugs for breast diseases.

### Description of Drawings

Figures 1A-1G are graphs for illustrating the effects of different concentrations of additive components in the culture medium on the *in vitro* proliferation of primary mammary epithelial cells.

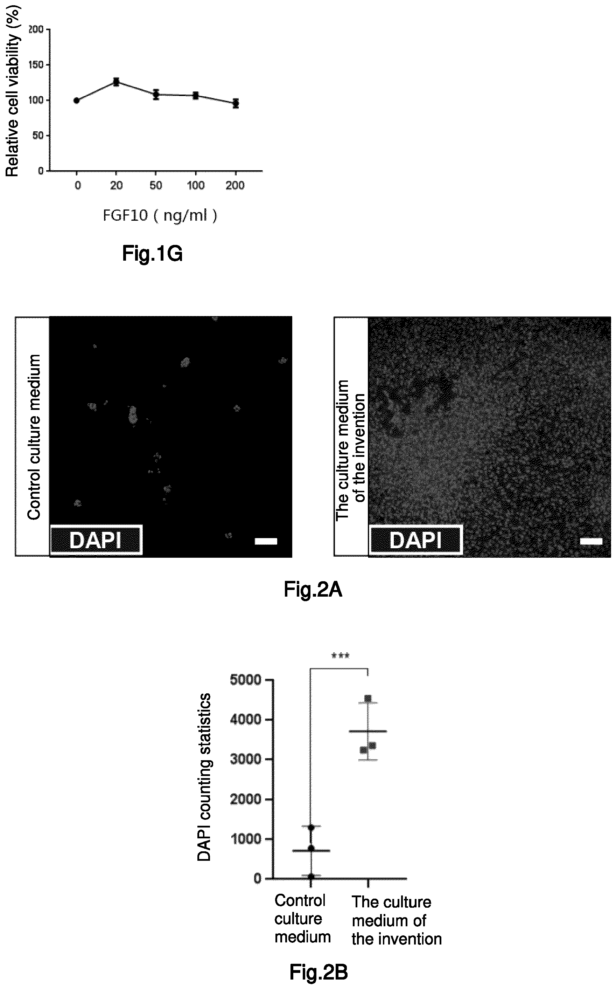

Figure 2A shows the results of fluorescent staining of the nuclei of primary mammary epithelial cells (under a 100x microscope), which were obtained by culturing the primary breast tumor cells isolated from a clinical tissue sample of breast cancer to the third passage using the culture medium for mammary epithelial cells of the invention and the control culture medium in parallel, and then continuing to culture for 14 days; Figure 2B shows the results of fluorescent labeling (DAPI) and statistical analysis of the cell nuclei of the cells obtained by culturing primary cells derived from three different breast tumor patients in parallel using the same culture method as in Figure 2A, and "***" indicates P<0.001.
Figures 3A and 3B are a comparison graph and a curve graph of culture days - population doubling number, showing the effects of continuous culture of a case of primary mammary epithelial cells using the culture medium of the invention and the control culture medium, respectively.
Figures 4A and 4B are a comparison graph and a curve graph of culture days - population doubling number, showing the effects of continuous culture of another case of primary mammary epithelial cells using the culture medium of the invention and the control culture medium, respectively.
Figure 5 shows the results of DAPI fluorescent labeling, immunofluorescence staining with biomarkers specific for luminal epithelial cells (CK8) and myoepithelial cells (CK14), and multi-channel fluorescence signal overlap imaging (MERGE) of the mammary epithelial cells (under a 100x microscope), which were obtained by culturing the primary breast cancer cells isolated from a clinical tissue sample of breast cancer to the third passage using the culture medium for mammary epithelial cells of the invention and the control culture medium in parallel, and then continuing to culture for 14 days.
Figures 6A-6F are dose-effect curves of different drugs on breast tumor cells cultured with the culture medium for mammary epithelial cells of the invention.

### Detailed Description of the Invention

### [Example 1]

Isolation of human primary mammary epithelial cells and optimization of culture medium for mammary epithelial cells

### (1) Isolation of human primary mammary epithelial cells

The commercially available penicillin-streptomycin dual antibody solution (Corning, containing 10,000 U/ml penicillin and 10 mg/ml streptomycin) was added to DMEM/F12 culture medium (manufactured by Corning) at a volume ratio of 2% for transportation and washing of samples, hereinafter referred to as "transport fluid".

The breast tumor tissue samples, 1#, 2#, 3#, 4#, and 5#, were derived from five breast tumor patients' surgically removed cancer tissue samples, who had given explanation and consent. One (1#) of the samples is used for explanation below. The above tissue samples were collected within half an hour of surgical resection of the patient. More specifically, under a sterile environment, tissue samples with a volume of 0.5 cm³ or more were cut from non-necrotic areas, placed in pre-cooled transport fluid, and transported to the laboratory on ice.

In the biosafety cabinet, the tissue sample (1#) was transferred to a 100 mm cell culture dish. The tissue sample was rinsed with the transport fluid. The blood cells on the surface of the tissue sample was washed away. Unwanted tissues such as skin and fascia on the surface of the tissue sample were removed.

The rinsed tissue sample was transferred to another new 100 mm culture dish; 10 mL of transport fluid was added, and a sterile scalpel blade and a forceps were used to divide the tissue sample into tissue fragments less than 1 mm³ in diameter.

The tissue sample fragments were transferred into a 50 mL centrifuge tube, and centrifuged at 1200 rpm for 5 minutes using a tabletop centrifuge; after the supernatant was carefully removed from the centrifuge tube with a pipettor, the precipitate was resuspended in 10 mL serum-free DMEM/F12 culture medium containing collagenase II (1 mg/mL) (manufactured by Sigma) and collagenase IV (1 mg/mL) (Sigma), and placed in a constant temperature shaker of 37°C for shake digestion for 30 - 90 minutes; then it was centrifuged at 350 g/minute in a tabletop centrifuge for 5 minutes; the supernatant was discarded, and the digested tissue cells were resuspended in 10 mL of DMEM/F12 culture medium containing 10% fetal bovine serum (Gibco), ground and sieved, and the pore size of the cell sieve was, for example, 100 µm; the sieved cell suspension was collected in a 50 mL centrifuge tube; and the cells were counted with a hemocytometer.

The cell suspension was then centrifuged at 350 g/minute for 5 minutes in a centrifuge; after the supernatant was discarded, the pellet was resuspended in the Basal Medium as described below.

Another four breast tumor tissue samples were isolated according to the same process as above.

### (2) Coating of cell culture plates

Extracellular matrix gel (Matrigel, manufactured by Corning) was diluted with DMEM/F12 at a ratio of 1:50 to prepare an extracellular matrix gel diluent. 250 µl/well of the extracellular matrix gel diluent was added to a 24-well culture plate to completely cover the bottom of the culture plate wells. After standing in a 37°C incubator for 1 hour, the extracellular matrix gel diluent was removed, and a culture plate coated with extracellular matrix gel was obtained.

### (3) Screening of additive factors in the culture medium of primary mammary epithelial cells

First, a basal culture medium based on WO 2021/088119 was prepared. To a commercially available DMEM/F-12 culture medium, GlutaMAX-I (manufactured by Thermo Fisher Scientific) was added at the concentration specified in the instruction manual (1:100 dilution), human insulin (Sigma) was added at a final concentration of 10 µg/ml, ROCK inhibitor Y27632 (Sigma) was added at a final concentration of 10 µM, penicillin-streptomycin (Thermo Fisher Scientific) was added at a 1:100 dilution, human amphiregulin (manufactured by R&D systems) was added at a final concentration of 20 ng/ml, epidermal growth factor (EGF, manufactured by Peprotech) was added at a final concentration of 10 ng/ml, B27 (Thermo Fisher Scientific) was added at a 1:50 dilution, human neuregulin 1 (Peprotech) was added at a final concentration of 10 nM, fibroblast growth factor 7 (FGF7, R&D systems) was added at a final concentration of 10 ng/ml), and TGFβ1 inhibitor A8301 (manufactured by MCE) was added at a final concentration of 500 nM, to prepare a basal culture medium for primary mammary epithelial cells, hereinafter referred to as "Basal Medium".

Then, different types of additives (Table 1) were added to the Basal Medium to prepare culture mediums for mammary epithelial cells containing different additives. The culture medium with different components was added to a 24-well plate coated with extracellular matrix gel at a volume of 500 µl/well, and 3 replicates were prepared for each culture medium formulation. The breast cancer cells (1#) isolated from the breast tumor tissue in Example (1) were inoculated at a cell density of 3×10⁴ cells/well in a 24-well culture plate coated with extracellular matrix gel, and cultured at 37°C and 5% CO₂ concentration using different culture medium formulations, respectively. The culture medium was replaced every 3 days after the start of culture. After 14 days of culture, the cells were counted. Basal Medium without addition of any additive shown in Table 1 was used as an experimental control.

The tumor cells isolated from the rest four cases of breast tumor tissue samples were also cultured and counted in the same manner as above.

The statistical results are shown in Table 1.

**[Table 1]**

| Number | Medium additive | Supplier | Concentration | Level of promoting proliferation |
|---|---|---|---|---|
| 1 | insulin-like growth factor 1 IGF1 | Peprotech | 50ng/ml | + |
| 2 | basic fibroblast growth factor bFGF | R&D | 50ng/ml | ++ |
| 3 | β-estradiol | Sigma | 1nM | ○ |
| 4 | fibroblast growth factors □ 10 FGF10 | R&D | 100ng/ml | + |
| 5 | tumor necrosis factor α TNF-α | Novoprotein | 10ng/ml | ++ |
| 6 | sodium pyruvate | ThermoFisher | 1mM | ○ |
| 7 | non-essential amino acid(s) MEM-NEAA | ThermoFisher | 100X | ○ |
| 8 | combination of above additives | - | - | ++++ |

Wherein, "+" indicates that compared with the Basal Medium, the culture medium added with the additive(s) has the effect of promoting the proliferation of at least three cases of primary breast cancer cells isolated from breast tumor tissue; the number of "+" indicates the degree of proliferation promotion, "-" indicates that the culture medium added with the additive(s) has the effect of inhibiting the proliferation of at least two cases of primary breast cancer cells isolated from breast tumor tissue; "o" indicates that the culture medium added with the additive(s) has no significant effect on the proliferation of at least three cases of primary breast cancer cells isolated from breast tumor tissue.

The results showed that several additives, including insulin-like growth factor 1, basic fibroblast growth factor, fibroblast growth factor 10, and tumor necrosis factor α, could promote the growth of at least three cases of primary breast tumor cells isolated from breast tumor tissue, among the additives, the effects of basic fibroblast growth factor and tumor necrosis factor α were stronger. When all the above factors were added to the Basal Medium, the degree of proliferation promotion was significantly increased.

### (4) Optimization of the concentration of additives in the culture medium for human mammary epithelial cells

The following 7 different formulations of culture mediums were further prepared:
Formulation 1: the Basal Medium was added with 1:1 00-fold diluted MEM-NEAA, 1 mM sodium pyruvate, 50 ng/ml IGF1, 20 ng/ml bFGF, 10 ng/ml TNF-α and 20 ng/ml FGF10;
Formulation 2: the Basal Medium was added with 10 nM β-estradiol, 1 mM sodium pyruvate, 50 ng/ml IGF1, 20 ng/ml bFGF, 10 ng/ml TNF-α and 20 ng/ml of FGF10;
Formulation 3: the Basal Medium was added with 10 nM β-estradiol, 1:100-fold diluted MEM-NEAA, 50 ng/ml IGF1, 20 ng/ml bFGF, and 10 ng/ml TNF-α and 20 ng/ml FGF10;
Formulation 4: the Basal Medium was added with 10 nM β-estradiol, 1:100-fold diluted MEM-NEAA, 1 mM sodium pyruvate, 20 ng/ml bFGF, and 10 ng/ml TNF-α and 20 ng/ml FGF10;
Formulation 5: the Basal Medium was added with 10 nM β-estradiol, 1:100-fold diluted MEM-NEAA, 1 mM sodium pyruvate, 50 ng/ml IGF1, and 10 ng/ml TNF-α and FGF10 at 20 ng/ml;
Formulation 6: the Basal Medium was added with 10 nM β-estradiol, 1:100-fold diluted MEM-NEAA, 1 mM sodium pyruvate, 50 ng/ml IGF1, 20 ng/ml bFGF and 20 ng/ml FGF10;
Formulation 7: the Basal Medium was added with 10 nM β-estradiol, 1:100-fold diluted MEM-NEAA, 1 mM sodium pyruvate, 50 ng/ml IGF1, 20 ng/ml bFGF and 10 ng/ml of TNF-α.

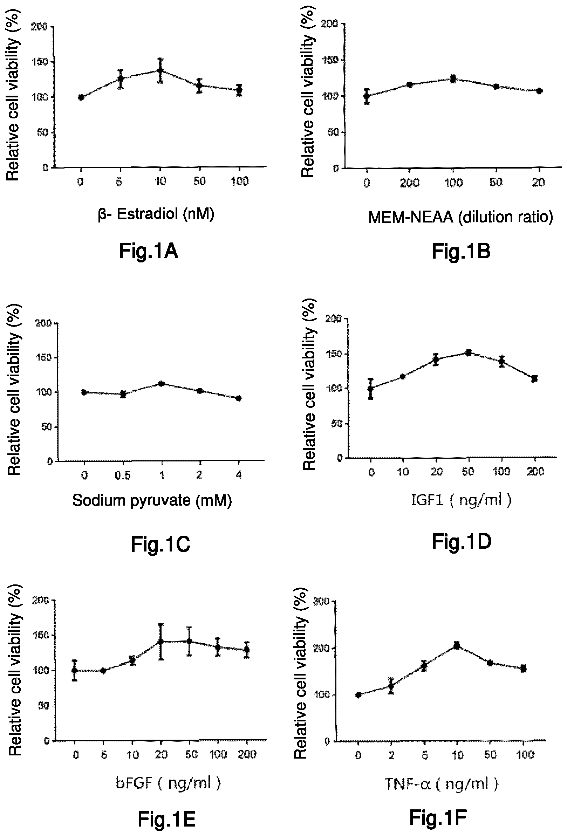

Subsequently, β-estradiol (Sigma) was added to Formulation 1 to prepare culture mediums for human mammary epithelial cells containing β-estradiol at final concentrations of 5 nM, 10 nM, 50 nM, and 100 nM, and each culture medium was added to a 96-well plate coated with Matrigel at a volume of 100 µl/well, with 3 replicates for each concentration. The primary breast cancer cells (2#) isolated from breast tumor tissue in Example (1) were seeded in a Matrigel-coated 96-well culture plate at a density of 500 cells/well and cultured in Formulation 1 containing different concentrations of β-estradiol at 37°C and 5% CO₂. The culture medium was replaced every 3 days after the start of culture. On the 14th day of culture, the cells were counted. Formulation 1 was used as an experimental control, i.e., the content of β-estradiol was 0 nM. The results are shown in Figure 1A.

Non-essential amino acids (MEM-NEAA) additive (Thermo Fisher) was added to Formulation 2 at a dilution ratio of 1:200, 1:100, 1:50, and 1:20 to prepare culture mediums for human mammary epithelial cells containing different concentrations of non-essential amino acids. The culture mediums of different formulations were added to a 96-well plate coated with Matrigel at a volume of 100 µl/well, with 3 replicates for each concentration. Primary cells were cultured and counted in the same manner as above. As an experimental control, Formulation 2 was used, i.e., the content of non-essential amino acids was 0. The results are shown in Figure 1B.

Similarly, sodium pyruvate (Thermo Fisher) was added to Formulation 3 to prepare culture mediums for human mammary epithelial cells containing sodium pyruvate at final concentrations of 0.5 mM, 1 mM, 2 mM, and 4 mM. The culture mediums of different formulations were added to a 96-well plate coated with Matrigel at a volume of 100 µl/well, with 3 replicates for each concentration. Primary cells were cultured and counted in the same manner as above. As an experimental control, Formulation 3 was used, i.e., the content of sodium pyruvate was 0. The results are shown in Figure 1C.

Then, insulin-like growth factor 1 (IGF1, manufactured by R&D) was added to formulation 4 to prepare culture mediums for human mammary epithelial cells containing IGF1 at final concentrations of 10 ng/ml, 20 ng/ml, 50 ng/ml, 100 ng/ml, and 200 ng/ml, and the culture mediums containing different concentrations of IGF1 were added to a 96-well plate coated with Matrigel at a volume of 100 µl/well, with 3 replicates for each concentration. Primary cells were cultured and counted in the same manner as above. As an experimental control, Formulation 4 was used, i.e., the content of IGF1 was 0. The results are shown in Figure 1D.

Similarly, culture mediums for human mammary epithelial cells containing basic fibroblast growth factor (bFGF, manufactured by R&D) at different concentrations were prepared using Formulation 5, and the final concentrations of bFGF were 5 ng/ml, 10 ng/ml, 20 ng/ml, 50 ng/ml, 100 ng/ml, and 200 ng/ml, respectively. Primary cells were cultured and counted in the same manner as above. As an experimental control, Formulation 5 was used, i.e., the content of bFGF was 0. The results are shown in Figure 1E.

Then, culture mediums for human mammary epithelial cells containing tumor necrosis factor-α (TNF-α, manufactured by Novoprotein) at different concentrations were prepared using Formulation 6, and the final concentrations of TNF-α were 2 ng/ml, 5 ng/ml, 10 ng/ml, 50 ng/ml, and 100 ng/ml, respectively. Primary cells were cultured and counted in the same manner as above. As an experimental control, Formulation 6 was used, i.e., the content of TNF-α was 0. The results are shown in Figure 1F.

Finally, culture mediums for human mammary epithelial cells containing fibroblast growth factor 10 (FGF10, manufactured by R&D) at different concentrations were prepared using Formulation 7, and the final concentrations of FGF10 were 20 ng/ml, 50 ng/ml, 100 ng/ml, and 200 ng/ml, respectively. Primary cells were cultured and counted in the same manner as above. As an experimental control, Formulation 7 was used, i.e., the content of FGF10 was 0. The results are shown in Figure 1G.

The results show that different concentrations of additives had a dose-dependent pro-proliferation effect on the *in vitro* proliferation of primary breast cancer cells within a certain concentration range. Wherein, β-estradiol, IGF1, bFGF, and TNF-α started to show obvious proliferative effects when added at lower concentrations. Furthermore, the preferred concentration of β-estradiol was 5 nM to 50 nM, more preferably 5 nM to 10 nM; the preferred concentration of IGF1 was 10 ng/ml to 200 ng/ml, more preferably 20 ng/ml to 100 ng/ml; the preferred concentration of bFGF was 10 ng/ml to 200 ng/ml, more preferably 20 ng/ml to 100 ng/ml; the preferred concentration of TNF-α was 2 ng/ml to 100 ng/ml, more preferably 5 ng/ml to 50 ng/ml. In addition, the additive FGF10 also had a dose-dependent pro-proliferation effect on the *in vitro* proliferation of primary breast cancer cells within a certain concentration range, and its preferred addition concentration is 0 ng/ml~50 ng/ml.

### (5) Study on the proliferation promoting effect of culture medium for human mammary epithelial cells

Two different formulations of mediums were further prepared as follows:
1. Control culture medium: the preparation procedure referred to WO 2021/088119, i.e., a culture medium containing the following components was prepared: DMEM/F-12 culture medium (Corning) + 1:100 volume ratio of GlutaMAX-I (Thermo Fisher SCIENTIFIC) + 10 µg/ml human insulin (Sigma) + 10 µM Y27632 (Sigma) + 1:100 volume ratio of penicillin-streptomycin (Thermo Fisher SCIENTIFIC) + 20 ng/ml human amphiregulin (R&D systems) + 10 ng/ml EGF (Peprotech) + 1:50 volume ratio of B27 (Thermo Fisher SCIENTIFIC) + 10 nM human neuregulin 1 (Peprotech) + 10 ng/ml FGF7 (R&D systems) + 500 nM A8301 (MCE) + 500 nM SB202190;
2. Complete culture medium (hereinafter referred to as "the culture medium of the invention"): 10 nM β-estradiol, 20 ng/ml bFGF, 50 ng/ml IGF1, and 10 ng/ml TNF-α were added to the Basal Medium.

The proliferative effect of the control culture medium and the culture medium of the invention on primary breast cancer cells *in vitro* was compared. The control culture medium and the culture medium of the invention were added to a 24-well plate coated with Matrigel at a volume of 500 µl/well, with 3 replicates per group.

According to the method of Section (1) of Example 1, breast cancer cells (1#~3#) were isolated from breast tumor tissues and inoculated in Matrigel-coated 24-well culture plates at a cell density of 3×10⁴ cells/well, respectively, and cultured using two different culture medium formulations at 37°C and 5% CO₂. The cells were subcultured to the third passage at an equal inoculation density and then cultured until the 14th day. The breast cancer cells cultured using different culture medium formulations were subjected to nuclear fluorescence staining and counting. Specifically, each group of cells was rinsed twice with PBS buffer, fixed with 4% paraformaldehyde for 15 minutes, and then incubated with TBST (TBS (manufactured by Sangon Biotech (Shanghai)) + 0.1% Tween 20) containing 1% BSA (Sangon Biotech (Shanghai)) and 1% Triton X-100 (Sangon Biotech (Shanghai)) at room temperature for 1 hour, and then rinsed with TBST buffer 3 times, each for 3 minutes. The TBST solution was removed. The cells were incubated with 1 µg/mL DAPI dye (Sigma) for 10 minutes. The cells were rinsed once with PBS. Random field shots were taken under a 100x fluorescence microscope after the coverslip were sealed with a drop of mounting medium (Thermo Fisher Scientific).

Representative results are shown in Figures 2A and 2B. Figure 2A is a microscopic photograph of 2# breast cancer cells after continuous culture to the third passage with control culture medium and the culture medium of the invention, and then cultured at a density of 3×10⁴ cells/well for 14 days. Figure 2B shows the statistics of DAPI counting results for breast cancer cells (1#∼3#) derived from different patients using the same procedure as 2#. The vertical axis is the statistics of counting results for DAPI staining under random fields of view. As shown in Figure 2A and Figure 2B, compared with the control culture medium, the number of cells cultured to the third passage using the culture medium of the invention can continue to increase by about 5.2 times within 14 days.

### [Example 2]

Continuous *in vitro* culture of human mammary epithelial cells and comparison with existing culture methods

### (1) Culture of primary mammary epithelial cells derived from human breast tumor tissue

Using the same method as section (1) of Example 1, primary breast cancer cells (6#, 7#) were isolated from cancer tissues of two breast tumor patients. Then, the isolated breast cancer cells were counted using a Hemocytometer, and then cells of 6# were inoculated into two 6-well plates coated with Matrigel (Corning) at the same density (2×10⁵ cells/well) in parallel. The coating method was as follows: Matrigel was diluted with DMEM/F12 culture medium at a ratio of 1:50 to prepare an extracellular matrix diluent, 1.5 ml/well of the extracellular matrix gel diluent was added to the 6-well culture plates to completely cover the bottom of the culture plate wells, and after standing in a 37°C incubator for 1 hour, the extracellular matrix gel diluent was removed, and culture plates coated with extracellular matrix were obtained.

3 mL/well of the control culture medium prepared in section (5) of Example 1 and the culture medium of the invention were respectively added to culture wells of two 6-well culture plates coated with extracellular matrix, and cultured at 37°C with a CO₂ concentration of 5%. The culture medium was replaced every 3 days after the start of culture.

The freshly isolated primary breast cancer cells #7 was subjected to the same procedure as #6.

### (2) Comparison of the effects of continuous in vitro culture using different culture mediums for human mammary epithelial cells

When the human breast cancer cells in the culture plate grew to cover about 80% of the bottom area, the supernatant of culture medium in the original 6-well plate was discarded. The cells were digested by adding 1mL of 0.05% trypsin (Thermo Fisher: 25300062) and incubated at 37 °C for 10 to 20 minutes. After the cells were completely digested, the digested cells were resuspended with 5 mL of DMEM/F12 culture medium containing 10% (v/v) fetal bovine serum, 100 U/mL penicillin and 100 µg/mL streptomycin and collected into a centrifuge tube, and centrifuged at 300 g/min for 5 minutes. The cell pellet after centrifugation was resuspended with the control culture medium or the culture medium of the invention, and cell counting was performed on the cell suspension using a counting chamber. The cells of each group were seeded in another 12-well culture plate coated with Matrigel at a passage ratio of 1:10 and continued to be cultured.

When the cells after passage grew to cover about 80% of the bottom area in the culture plate again, the cultured cells were digested, collected and counted again by the operation method described above. The cells were also inoculated at a 1:10 ratio and cultured continuously.

The primary breast cancer cells of 7# were repeated in the same way as 6#.

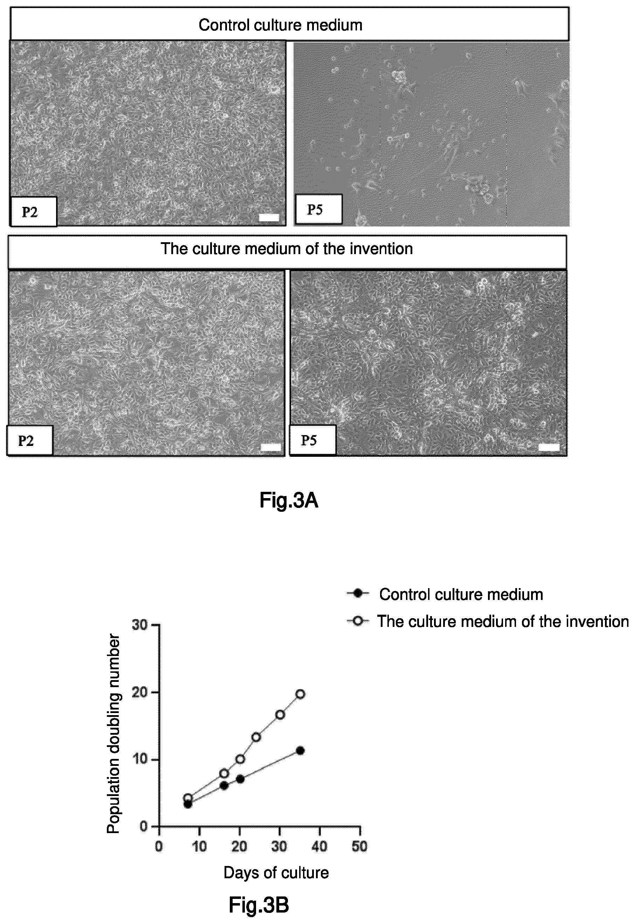

Figure 3A shows cell photographs taken under a 100x phase contrast microscope of 6# cells cultured to the second and fifth passages using the control culture medium and the culture medium of the invention, respectively.

Figure 3B is a continuous growth curve of cells of 6# under the culture conditions of the culture medium of the invention and the control culture medium, drawn using Graphpad Prism7.0 software, with the culture days as the horizontal axis and the cell population doubling number as the vertical axis.

The following is the calculation formula for the cell population doubling number of primary mammary epithelial cells under different technical culture conditions:
Cell population doubling number = [log (N/X₀)]/log2
wherein, N is the number of cells at passage, and X₀ is the number of cells at the initial inoculation (see Greenwood et al., Environ Mol Mutagen 2004, 43 (1): 36-44).

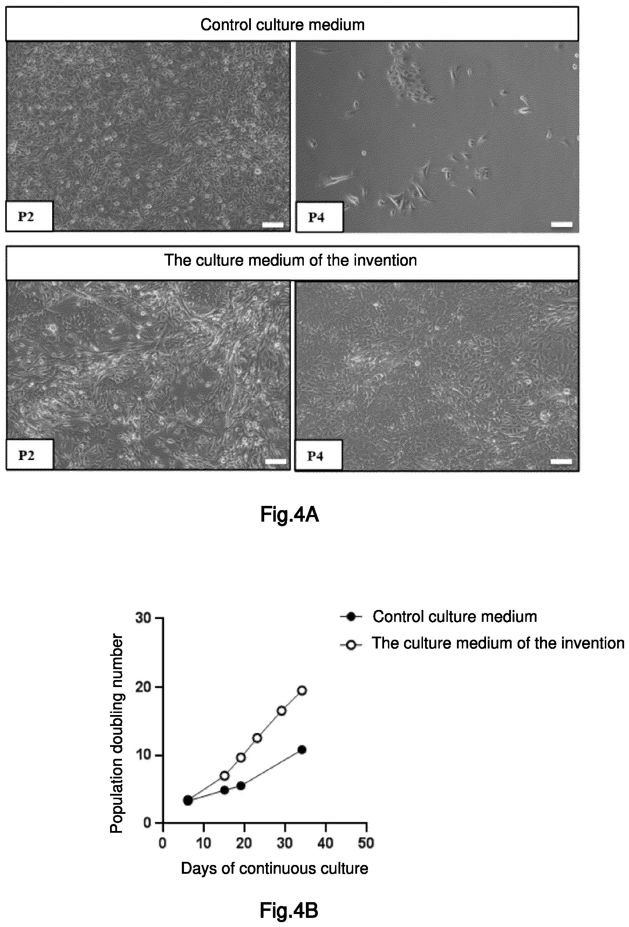

Figure 4A shows cell photographs of cells of 7# cultured to the second and fourth passages after continuous culture using the control culture medium and the culture medium of the invention, respectively, according to the same operating steps. Figure 4B is a curve chart of the culture days-population doubling number of cells of 7# under the culture conditions of the culture medium of the invention and the control culture medium, drawn using Graphpad Prism7.0 software.

It can be confirmed from Figures 3A, 3B and Figures. 4A, 4B that breast cancer cells cultured using the culture medium of the invention can proliferate sustainably, and the proliferation rate is significantly better than that of the known control culture medium.

### [Example 3]

Identification of immune markers of mammary epithelial cells
(1) The control culture medium and the culture medium of the invention were prepared according to the description of section (5) of Example 1.
(2) Cancerous tissue of about the size of a soybean grain was taken from a clinical surgical resection sample of a breast cancer patient, and primary breast cancer cells (3#) were isolated and obtained using the same method as section (1) of Example 1. Primary breast cancer cells (3#) were cultured to the third passage using the methods in section (1) and (2) of Example 2 in parallel, respectively.
(3) The expression of important biomarkers related to cancer on human breast cancer cells was detected by immunofluorescence.

The primary antibodies used in this experiment were CK8 (manufactured by Abcam) and CK14 (Abcam). For CK8, the secondary antibody Anti-Mouse IgG (H+L), F(ab')2 Fragment (Alexa Fluor^{®} 488 Conjugate) (manufactured by Cell Signaling Technology) was used, and for CK14, the secondary antibody Anti-Rabbit IgG (H+L), F(ab')2 Fragment (Alexa Fluor^{®} 594 Conjugate) (Cell Signaling Technology) was used. Wherein, CK8 is an important biomarker for breast cancer, generally expressed on the luminal epithelial cells of breast tumors; CK14 is a recognized important biomarker for myoepithelial cells of breast tumors. In clinical practice, CK8 and CK14 are often used in the differential diagnosis of breast cancer.

Specifically, the supernatant of the culture medium in the original 6-well plate was discarded, and 1 mL of 0.05% trypsin (Thermo Fisher) was added to digest the cells, and after incubation at 37°C for 15 minutes, the digested cells were resuspended with 5 mL of DMEM/F12 culture medium containing 10% (v/v) fetal bovine serum, 100 U/mL penicillin and 100 µg/mL streptomycin and collected in a centrifuge tube, and centrifuged at 300 g/minutes for 5 minutes. The cell pellets of each group after centrifugation were resuspended using the culture medium of the invention and the known control culture medium, respectively. Cell counting was performed on the cell suspension using a counting chamber. The cells were inoculated on coverslips coated with Matrigel at a density of 4×10⁴ cells/coverslip, and the coating method was the same as section (2) of Example 1. The cells of each group were cultured using the culture medium of the invention and the known control culture medium, respectively, so that the cells grew on the coverslips.

After 14 days of culture, the cells were rinsed twice with PBS buffer, fixed with 4% paraformaldehyde for 15 minutes, and then incubated with TBST (TBS + 0.1% Tween 20) containing 1% BSA (Sangon Biotech (Shanghai)) and 1% Triton X-100 for 1 hour at room temperature, and rinsed three times with TBST buffer for 3 minutes each time. After removing the TBST solution, 50 µL of primary antibody dilution solution (the CK8 antibody was diluted 1:100 times; the CK14 antibody was diluted 1:1000 times) was added to the slide, incubated at 4°C for 12-16 hours, and then rinsed three times with PBS for 3 minutes each time; the secondary antibody, Anti-Mouse IgG (H+L), F(ab')2 Fragment (Alexa Fluor^{®} 488 Conjugate) (8 µg/ml ) was added, incubated at room temperature for 60 minutes, and rinsed three times with PBS for 3 minutes each time. Subsequently, the secondary antibody, Anti-Rabbit IgG (H+L), F(ab')2 Fragment (Alexa Fluor^{®} 594 Conjugate) (8 µg/ml) was added, incubated at room temperature for 60 minutes, and rinsed three times with PBS for 3 minutes each time. The cells were incubated with 1 µg/mL DAPI dye (Sigma) for 10 minutes, and rinsed with PBS once. Photographs were taken under a fluorescence microscope at 100x magnification after coverslips were sealed with a drop of mounting medium (Thermo Fisher Scientific).

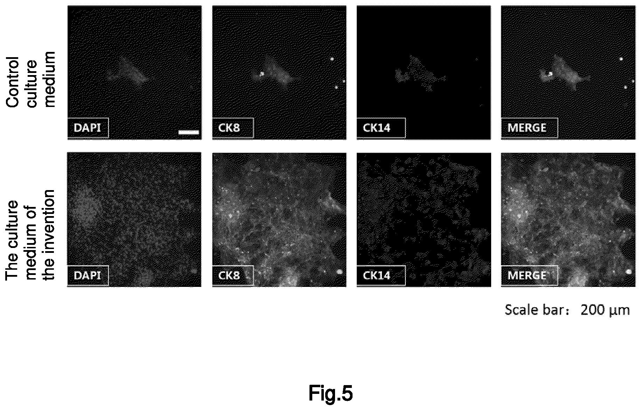

The results are shown in Figure 5, showing the results of immunofluorescence staining with biomarkers specific for luminal epithelial cells (CK8) and myoepithelial cells (CK14); in addition, DAPI fluorescent labeling is used to indicate the position and number of cell nuclei, and MERGE is used to indicate the imaging results produced by overlapping the three fluorescent staining labels of CK8, CK14 and DAPI.

According to Figure 5, a large number of cells highly expressing breast cancer cell-specific biomarkers CK8 and CK14 proteins can be cultured using the culture medium and culture method of the invention; while the primary breast cancer cells derived from the same sample cultured using the known control culture medium yielded only a small number of breast cancer cells, indicating that the culture technology of the invention can achieve the effect of efficiently culturing breast cancer cells.

### [Example 4]

### In vitro drug sensitivity test of breast cancer cells

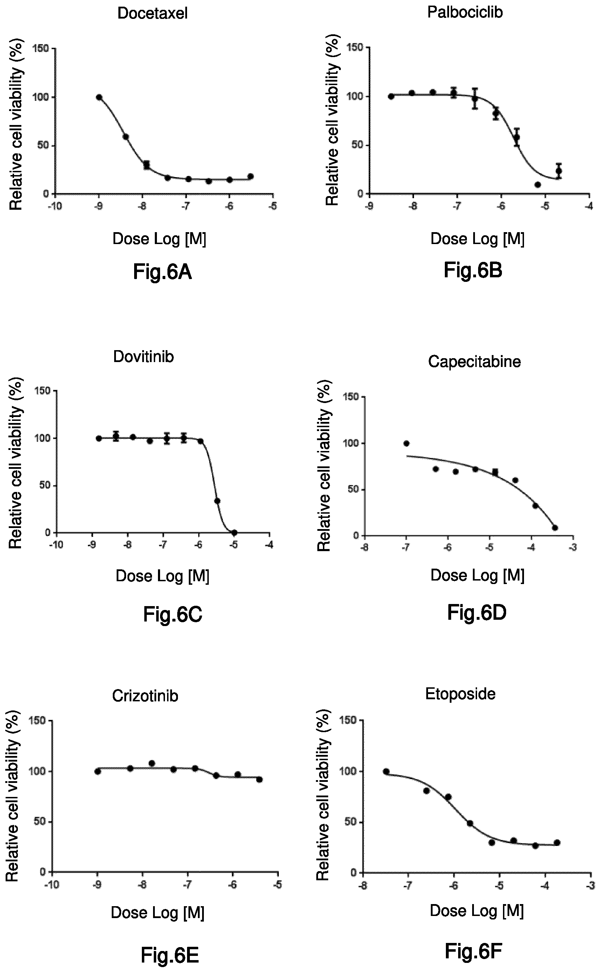

In the following, taking the surgical resection sample of breast tumor patient as an example, it shows that breast cancer cells cultured from patient-derived breast tumor tissue samples can be used to detect the sensitivity of tumor cells of patients to different drugs.
I. Plating of primary breast cancer cells: using the culture medium of the invention in section (5) of Example 1, breast tumor cells (5#) were cultured by the method described in section (1) of Example 2, and the cells were inoculated in a 384-well plate at a density of 3000 to 5000 cells/well, and the cells were subjected to adherent culture overnight.
II. Drug gradient experiment:
   (1) The drug storage plate was prepared by the concentration gradient dilution method: 10 µL of the drug stock solutions to be tested (the concentration of the drug stock solution was prepared at 2 times the maximum plasma concentration Cₘₐₓ of the drug in the human body) were respectively taken and added to a 0.5 mL EP tube containing 20 µL of DMSO, and then 10 µL was aspirated from the above EP tube into a second 0.5 mL EP tube containing 20 µL of DMSO, that is, the drug was diluted 1:3. The above process was repeated for gradually dilution until 8 or 9 concentrations required for dosing were obtained. Different concentrations of drugs were added to the 384-well drug storage plate. Equal volume of DMSO was added to each well of the solvent control group as a control. In this example, the drugs to be tested are clinically approved anti-tumor drugs, docetaxel (MCE), palbociclib (MCE), dovitinib (MCE), capecitabine (MCE), crizotinib (MCE) and etoposide (MCE).
   (2) Using a high-throughput automated workstation (purchased from Perkin Elmer), different concentrations of drugs and solvent controls in the 384-well drug storage plates were added to the 384-well cell culture plates plated with the breast tumor cells. Both the drug groups and the solvent control groups were set up with 3 replicate wells. The volume of drug added to each well was 100 nL.
   (3) Cell activity detection: 72 hours after drug administration, Cell Titer-Glo assay kit (manufactured by Promega) was used to detect the chemiluminescence value of the cultured cells after drug administration. The magnitude of the chemiluminescence value reflects the cell viability and the effect of the drug on the cell viability. The prepared Cell Titer-Glo detection solution was added to each well, and a microplate reader was used to detect the chemiluminescence value after mixing.
      Graphpad Prism 7.0 software was used to draw graphs and calculate the median inhibition concentration IC₅₀.
   (4) The results of drug sensitivity test are shown in Figures 6A-6F.

Figures 6A-6F show the drug sensitivity of breast tumor cells (5#) cultured from a surgically removed cancer tissue sample of a breast tumor patient to three targeted drugs, palbociclib, crizotinib and dovitinib, and three chemotherapy drugs, docetaxel, capecitabine and etoposide. The results show that cells from the same patient had different sensitivities to different drugs.

Wherein, breast cancer cells (5#) were more sensitive to docetaxel and etoposide, while they were less sensitive to capecitabine, crizotinib, dovitinib and palbociclib. This suggests that docetaxel and etoposide may be potential effective drugs for treating breast tumors in patient 5#.

The results of this example indicate the application potential of culturing patient-derived breast cancer cells with the culture medium for mammary epithelial cell of the invention and performing *in vitro* drug sensitivity testing for screening and predicting the efficacy of clinical drugs in patients with breast tumors.

### Industrial applicability

The invention provides a culture medium and a culture method for mammary epithelial cells, and the cultured cells can be used for drug efficacy evaluation and screening. Therefore, the invention is suitable for industrial application.

Although the invention has been described in detail above with general descriptions and specific embodiments, it is obvious to those skilled in the art that some modifications or improvements can be made to the invention. Therefore, these modifications or improvements made without departing from the spirit of the invention are within the protection scope claimed by the invention.

## Claims

1. A culture medium for culturing mammary epithelial cells, **characterized in that**,
comprising β-estradiol, insulin-like growth factor 1, basic fibroblast growth factor, tumor necrosis factor-α, and optionally fibroblast growth factor 10.

2. The culture medium of claim 1, **characterized in that** the amount of each component in the culture medium satisfies any one or more or all of the following conditions:
the concentration of β-estradiol is 5 nM to 50 nM;
the concentration of insulin-like growth factor 1 is 10 ng/ml to 200 ng/ml;
the concentration of basic fibroblast growth factor is 10 ng/ml to 200 ng/ml;
the concentration of tumor necrosis factor-α is 2 ng/ml to 100 ng/ml.

3. The culture medium of claim 1 or 2, **characterized in that** the amount of each component in the culture medium satisfies any one or more or all of the following conditions:
the concentration of β-estradiol is 5 nM to 10 nM;
the concentration of insulin-like growth factor 1 is 20 ng/ml to 100 ng/ml;
the concentration of basic fibroblast growth factor is 20 ng/ml to 100 ng/ml;
the concentration of tumor necrosis factor-α is 5 ng/ml to 50 ng/ml;
the concentration of fibroblast growth factor 10 optionally added is 0 ng/ml to 50 ng/ml.

4. The culture medium of any one of claims 1 to 3, **characterized in that** the culture medium further comprises amphiregulin, epidermal growth factor, insulin, B27, Y27632, neuregulin 1, fibroblast growth factor 7, A8301, and GlutaMAX-I.

5. The culture medium of claim 4, **characterized in that** the amount of each component in the culture medium satisfies any one or more or all of the following conditions:
the amount of amphiregulin is 10 ng/ml to 100 ng/ml, the amount of epidermal growth factor is 2.5 ng/ml to 20 ng/ml, the amount of insulin is 1 µg/ml to 10 µg/ml, B27 is added at a volume ratio of 1:25 to 1:100, the amount of Y27632 is 5 µM to 15 µM, the amount of neuregulin 1 is 5 nM to 20 nM, the amount of fibroblast growth factor 7 is 2.5 ng/ml to 20 ng/ml, the amount of A8301 is 100 nM to 500 nM, and GlutaMAX-I is added at a volume ratio of 1:50 to 1:200.

6. The culture medium of any one of claims 1 to 5, **characterized in that** the culture medium further comprises an initial culture medium selected from the group consisting of DMEM/F12, DMEM, F12 or RPMI-1640; and one or more antibiotic(s) selected from the group consisting of streptomycin/penicillin, amphotericin B and Primocin.

7. A culture method for culturing mammary epithelial cells, **characterized in that**, comprising a step of culturing primary mammary epithelial cells using the culture medium of any one of claims 1 to 6.

8. The culture method of claim 7, **characterized in that**, comprising the following steps:
(1) preparing the culture medium of any one of claims 1 to 6;
(2) coating a culture vessel with an extracellular matrix gel;
(3) inoculating primary mammary epithelial cells in the coated culture vessel and culturing them using the culture medium prepared in step (1).

9. A method for evaluating or screening drugs for treating breast diseases, **characterized in that**, comprising the following steps:
(1) obtaining primary mammary epithelial cells and culturing them using the culture method of claim 7 or 8;
(2) selecting the drug to be tested and preparing it into different concentration gradients;
(3) adding different concentrations of the drug prepared in step (2) into the mammary epithelial cells cultured in step (1); and
(4) detecting the cell viability.
